(19) Europäisches Patentamt
European Patent Office
Office européen des brevets

(11) Publication number: **0 002 192**
**B1**

(12) **EUROPEAN PATENT SPECIFICATION**

(45) Date of publication of patent specification: **04.11.81**

(21) Application number: **78101295.0**

(22) Date of filing: **02.11.78**

(51) Int. Cl.[3]: **C 12 P 19/40**, C 07 H 19/16 //A61K31/70, C12N9/10

(54) **Enzymatic synthesis of purine arabinonucleosides.**

(30) Priority: **03.11.77 GB 4566877**

(43) Date of publication of application: **13.06.79 Bulletin 79/12**

(45) Publication of the grant of the European patent: **04.11.81 Bulletin 81/44**

(84) Designated Contracting States: **CH DE FR GB**

(56) References cited:

**GB - A - 1 159 290**

**CHEMICAL ABSTRACTS, vol. 68, 1968, Columbus, Ohio, USA, A. IMIDA et al. "Ribosyl and deoxyribosyl transfer by bacterial enzyme systems", abstract no. 9666h**

(73) Proprietor: **THE WELLCOME FOUNDATION LIMITED**
**183-193 Euston Road**
**London NW1 2BP (GB)**

(72) Inventor: **Krenitsky, Thomas Anthony**
**106 Laurel Hill Road**
**Chapel Hill North Carolina 27514 (US)**
Inventor: **Elion, Gertrude Belle**
**Oxford Apartments 1 Banbury Lane**
**Chapel Hill North Carolina 27514 (US)**
Inventor: **Rideout, Janet Elizabeth**
**3101 Morningside Drive**
**Raleigh North Carolina 27607 (US)**

(74) Representative: **Berg, Wilhelm, Dr. et al,**
**Dr. Berg, Dipl.-Ing. Stapf, Dipl.-Ing. Schwabe, Dr. Dr. Sandmair Patentanwälte Postfach 860245**
**D-8000 München 86 (DE)**

**EP 0 002 192 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Enzymatic synthesis of purine arabinonucleosides

This invention relates to a process for preparing purine arabinonucleosides and more particularly, relates to a novel process for the enzymatic synthesis of the arabinonucleosides of purine and substituted purine derivatives.

The arabinonucleosides of purines, especially those of adenine and 2,6-diaminopurine, are useful and valuable as antiviral agents, but means hitherto known for their production have involved either somewhat complex chemical processes or difficult fermentation procedures. Although the production of ribonucleosides and 2'-deoxyribonucleosides of certain purines and purine analogues from naturally occurring purine nucleosides, purine nucleotides, pyrimidine nucleosides, and pyrimidine nucleotides have been described (Imada and Igarasi, *Ann. Rep. Takeda Res.,* 1966, *25;* Krenitsky *et al, J. Biol. Chem.,* 1967, *242,* 2675; Sivadjian *et al., Physiol. Veg.,* 1969, *7,* 31; Kaminura *et al., Agr. Biol. Chem.,* 1973, *37,* 2063, Holquin *et al., Eur. J. Biochem.,* 1975, *54,* 515; Stout *et al.,* Methods in Carbohydrate Chemistry, Whistler and Miller, Eds. *Vol. VII,* p. 19, Academic Press, N.Y., 1976; U.S. Patents No. 3,269,917 and No. 3,135,666), no such enzymatic synthesis of purine arabinonucleosides has been reported. Furthermore, it has been reported that one of the enzymes used in the present invention, purine nucleoside phosphorylase, is not capable of utilising arabinonucleosides as substrates (Doskocil and Holy. *Coll. Czechoslov. Chem. Commun.,* 1977, *42,* 370). For the sake of completeness, reference is also made to UK Patent Specification No. 1,159,290 and the article by Imada *et al* in *J. Bacteriol.* (1967), *94(5)* at pp. 1551—9, which form part of the technological background.

According to the present invention, there is provided a process for producing 9—B—D-arabinonucleosides of purines which comprises reacting a purine base of formula (I):

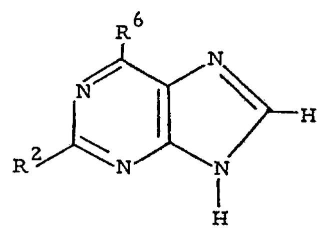

(I)

wherein a) $R^2$ is a hydrogen atom or an amino group and $R^6$ is a hydrogen or halogen atom or a hydroxyl, mercapto, amino, alkoxyl, alkylthio, aryl (lower) allkylthio or an alkylamino group; or b) $R^2$ is a fluorine atom and $R^6$ is an amino group; or c) $R^2$ is a methylamino group and $R^6$ is an amino or methylamino group; with arabinose-1-phosphate, in the presence of an arabinosyl transferring enzyme system of the phosphorylase type, and recovering the purine arabinonucleoside of formula (II) thus produced,

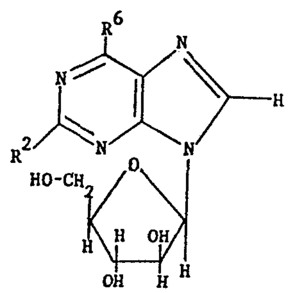

(II)

wherein $R^2$ and $R^6$ are as hereinbefore defined, from the reaction medium.

As used herein the term "alkyl" is denoted to mean an alkyl group having one to twelve carbon atoms in a straight or branched chain. The term "(lower) alkyl" is defined as an alkyl group having one to seven carbon atoms in a straight or branched chain.

A particular advantage of the above enzymatic synthesis is that whilst traditional methods employed in organic chemistry for making these compounds often yield products of doubtful stereochemical purity or configuration, the process of the present invention assures that the arabinosyl moiety of the product is in the correct position and of the correct stereochemistry, that is 9—B—D-arabinofuranosyl. In addition, the process of the present invention is easily adaptable to large-scale production.

The compounds of formula II are useful as antivirals, e.g. against vaccinia, herpes simplex, or rhinoviruses and may be administered *per se,* or as a pharmaceutically acceptable salt, topically, e.g. as an ointment, cream etc. at a dosage of 5 to 50 mg/kg of mammal body weight, e.g. mice, humans etc.,

or may be administered orally in a tablet at a dosage of 1 to 100 mg/kg of mammal body weight.

The antiviral activity of the arabinonucleosides of adenine and hypoxanthine ($R_2 = H$, $R^6 = NH_2$ or OH in formula II) has been documented in the literature (F. M. Schabel, Jr., Chemotherapy, *13*, 321 (1968)). The activity of several disubstituted purine arabinosides where $R_2 = NH_2$ $R_6 = NH_2$, OH, $NHCH_3$, or SH and where $R_2 = NHCH_3$, $R_6 = NH_2$ or $NHCH_3$ has also been reported (G. B. Elion, *et al.*, Ann. N.Y. Acad. Sci., 255, 468 (1975).

Some purine arabinosides of formula (II) have been reported as antitumor agents, e.g., where $R_2 = H$ and $R_6 = NH_2$, $NHCH_3$ or SH (J. J. Brink and G. A. LePage, Fed. Proc., *22*, 184 (1963); J. J. Brink and G. A. LePage, Can. J. Biochem. Physio., *43*, 1 (1965); A. P. Kimball, *et al.*, Can. J. Biochem. Physiol., *42*, 1753 (1964)) and where $R_2 = F$, $R_6 = NH_2$ (R. W. Brockman, *et al.*, The Pharmacologist, 19, 208 (1977)).

Furthermore, some of the purine arabinosides of formula (II) are useful as chemical intermediates, for example, $R_2 = H$, $R_6 = Cl$ was reacted with a variety of nucleophiles in order to prepare other purine arabinosides where $R_2 = H$; $R_6 = OCH_3$, $NHCH_3$, NHOH or $N(CH_3)_2$ and where $R_2 = H$; $R_6 = SH$ the compound was methylated or reacted with chlorine gas to give $R_2 = H$ and $R_6 = SCH_3$ and Cl, respectively. (E. J. Reist, *et al.*, J. Org. Chem., *27*, 3274 (1962)). When $R_2 = H$ or $NH_2$ and $R_6 = SH$, the compounds can be reacted with alkyl halides to give $R_2 = H$ or $NH_2$; $R_6$ = alkylthio which can be reacted with nucleophilic reagents such as metal alkoxides and amines. When $R_2 = H$ or $NH_2$ and $R_6 = SH$ the compounds can be reacted with alkylhalides and aryl(lower) alkyl halides to give the corresponding 6-alkylthio or aryl (lower) alkylthio compounds which can be reacted with nucleophilic reagents such as metal alkoxides and amines. When $R_2 = H$ or $NH_2$ and $R_6 = SH$, alkylthio, aryl (lower) alkylthio, the compounds can be reacted with reagents such as chlorine gas or hydrogen peroxide followed by reaction with a nucleophile in order to prepare a variety of compounds which are antiviral agents. The compounds of formula (II) wherein $R_6 = SH$, alkylthio or aryl (lower) alkylthio and $R_2 = H$ or $NH_2$ may be dethiolated with Raney nickel in water or ethanol at reflux temperature to produce the useful antiviral compounds of formula (II) where $R_2 = H$ or $NH_2$ and $R_6 = H$ as is conventional in the art. When $R_2 = H$ or $NH_2$ and $R_6$ = halogen, the halogen can be displaced by nucleophiles or hydrolysed in order to prepare active agents.

Although the arabinose-l-phosphate may be provided by synthetic processes known in themselves in the literature (Wright, R.S. and Khorana, H.G., *J. Am. Chem. Soc.*, *80*, 1994 (1958)), it can be convenient or even advantageous if the same is generated enzymatically *in situ* from an arabinosyl donor and an inorganic phosphate to obtain the required arabinose-l-phosphate. Although the reactions may be carried out separately that is, by isolating the arabinose-l-phosphate from an enzymatic reaction or chemically synthesizing arabinose-l-phosphate and using it as a starting material, it has been found advantageous to carry out both reactions in a "one-pot" process by forming the arabinose-l-phosphate intermediate *in situ*. The net effect of the coupled reactions therefore is the transfer of the arabinosyl moiety of the donor arabinonucleoside to the free purine base, thereby producing the desired arabinonucleoside.

The arabinosyl group donor may be a purine arabinonucleoside for example, adenine arabinonucleoside, a pyrimidine arabinonucleoside for example, uracil arabinonucleoside (see Examples II, III), a mixture of both types, or even a mixture of various arabinonucleosides and non-nucleoside material. However, for the purpose of the present invention it is preferable that the arabinosyl group donor is substantially free from non-nucleoside material and also that it is a pyrimidine arabinonucleoside.

The reasons for the preference for the use of a pyrimidine arabinonucleoside as the donor are two-fold. Firstly, the properties of the donor arabinonucleoside are sufficiently different from those of the desired product to facilitate easy purification. Secondly, the donor base liberated during the course of the reaction is a pyrimidine rather than a purine which results in substantially less competition between the donor base and the acceptor base for the catalytic site on the enzyme directly involved in product synthesis (purine nucleoside phosphorylase).

The pyrimidine arabinonucleoside donors may be prepared by any of the methods known in the art, for instance, according to the procedure described by Nishimura and Shimizu, *Chem. Pharm. Bull* (Tokyo), 1965, *13*, 803. Purine arabinonucleoside donors may be prepared by any of the methods known in the art for instance, according to the procedures described by Reist *et al.*, *J. Org. Chem.*, 1962, *27*, 3274.

It has been found that both reactions described hereinabove are catalysed by various enzymes which are present in many different microorganisms and mammalian tissues. The phosphorolysis of the donor arabinonucleoside is catalysed, for instance, by purine nucleoside phorphorylase if the donor is a purine arabinonucleoside, or by pyrimidine nucleoside phosphorylase, thymidine phosphorylase or uridine phosphorylase if the donor is a pyrimidine arabinonucleoside. The second reaction by which the desired purine arabinonucleoside is synthesised from the free base of formula (I) and arabinose-l-phosphate is catalysed by purine nucleoside phosphorylase.

The required arabinosyl transferring enzyme system therefore may consist of the latter phosphorylase alone, or in combination with any one of the former type, if the arabinosyl donor is an arabinonucleoside of a pyrimidine or pyrimidine analogue.

As previously stated, it has been found that the enzymes required for the catalysis of the reactions employed in the process of the present invention occur in many different microorganisms as well as mammalian tissues. For the purposes of the present invention, however, aerobic bacteria such as *B. stearothermophilus* and especially *E. coli* B, which is freely available from the American type culture collection under deposition No. ATCC 11303, were found to be excellent sources of such enzymes. The bacteria which provide the enzymes may be cultured under a variety of conditions. However with E. coli B media contained large quantities of glucose were found to be undesirable since the levels of the nucleoside phosphorylase enzymes in the bacterial cells were depressed in the presence of glucose.

It has been found that crude enzyme preparations are less suitable than purified preparations. This is due to the fact that crude preparations contain troublesome nucleic acids as well as enzymes other than those required for the process of the present invention. The extraneous enzymes in crude preparations catalyse undesirable alterations of substrates and products, and may even cause proteolysis of the required enzymes themselves. These factors decrease not only the yield of the desired products but also the ease with which they can be isolated from the reaction mixtures.

In most cases therefore, it is desirable to purify the crude enzyme preparations before addition to the reaction mixture. This may be achieved in a number of ways known in themselves in the art. For instance, the desired enzymes may be separated or concentrated from extracts of the cells by a combination of treatment with calcium phosphate gel and ion exchange chromatography. Alternatively the cell extract may be treated with streptomycin or nuclease (DNA ase + RNA ase) prior to calcium phosphate gel treatment or by nuclease (DNA ase + RNA ase) treatment prior to ion exchange chromatography. Nuclease treatment is particularly advantageous if performed under dialyzing conditions at 4° to 40°C, preferably at 25°C. Gel filtration has been found to be especially useful as a late or final step in the purification when only relatively small volumes of liquid are involved.

The enzymes, provided in a sufficiently effective state and concentration, may then be used to catalyse the aforementioned reactions. A typical reaction mixture contains an arabinosyl group donor, a purine base, inorganic phosphate, for example $K_2HPO_4$, and the appropriate enzyme or enzymes in an aqueous medium or in a medium containing up to 50% of an organic solvent such as methanol, ethanol, propanol, butanol, acetone, methylethylketone, ethylacetate, toluene, tetrahydrofuran, dioxane, dimethyl sulfoxide, trichloromethane, or Cellosolve. (The Word "Cellosolve" is a Trade Mark). The preferred concentration of organic solvent is 10%. The inorganic phosphate concentration can be from 0.001 mM to 2,000 mM, preferably 1 to 200 mM. The reaction is performed at near neutral pH, that is, in the pH range of about 5 to 9, preferably 6.0 to 8.5 and at a temperature of 3 to 70°C, preferably 25 to 45°C. Mild conditions are preferable since the glycosidic bond of purine arabinonucleosides is labile under acid conditions, particularly at elevated temperatures and the enzymes are unstable at extremes of temperature and pH. The preferable concentration of the enzymes is a function of the substrate efficiency of the particular arabinosyl donors and acceptors used and the length of time that one wishes to allow the reaction to proceed. With some substrates, for example when $R_2 = H$ and $R_6 = Cl$ in formula (II), it is preferable to use larger amounts of the enzyme in order to reduce the reaction time because of the instability of the purine base and product in aqueous solution. The purity of the enzymes used is a question of convenience. Crude extracts will catalyze the desired reactions but the yield of product is usually less and its isolation is more difficult than when purified enzymes are used for the reasons explained above. If the enzymes used are stored as ammonium sulfate suspensions, they are preferably added to the reactions as centrifuged pellets from the suspensions rather than as the whole suspensions.

Enzymes may be salvaged from reaction mixtures, for instance, by batch adsorption onto DEAE-cellulose after the reaction has reached a satisfactory point and subsequent removal from the soluble components of the reaction mixture by centrifugation or by gel filtration of reaction mixtures. In some cased, enzymes may be recycled by virtue of the fact that the bulk of the product precipitates out from the reaction mixture and upon its removal, more starting material may be added to the reaction fluid in order that product formation may be resumed. (See Example II).

Usually it is preferable that all the components are contained in suspension or solution but when highly soluble substrates are used an alternative procedure wherein a solution of the reaction mixture components except enzymes is pumped slowly through a column to which the appropriate enzymes have been fixed, (for instance, when the enzymes are adsorbed to a DEAE-cellulose column) may be preferable.

It has been found that, if it is so desired, enzymes may be conserved by allowing reactions to proceed for extended periods of time, for example, up to thirty days or longer. However, for reaction mixtures which are incubated for more than one day it is desirable to have an antimicrobial agent, for example sodium or potassium azide or toluene, in the reaction mixture, unless the reaction mixture is sterilised by filtration or some other technique known to the art.

The desired purine arabinonucleosides may be recovered or isolated by any of the known means for separating mixtures of chemical compounds into individual compounds. For example, the separation can be effected by utilizing differences in the solubilities in various solvents between the desired end product and impurities, the difference in their distribution coefficients between two solvent layers, the difference in their adsorbabilities to an adsorbent such as ion-exchange resins, the difference in their

rates of passage through cross-linked resins such as polyacrylamide gels, or the difference in their crystallizabilities from a solvent. In cases where the product crystallizes out of the reaction mixture, it can be collected by centrifugation or by filtration with or without a filter aid. In practice, these means for separation or isolation are carried out in combination or repeatedly depending on the desired purity and state of the products.

The following examples illustrate the invention but are not intended to limit it in any way.

## Example 1 — Enzyme Purification

*Assay Procedures*

The purification of the desired enzymes were monitored by the following assays, all of which were performed at 24°C.

Uridine phosphorylase activity was assayed spectrophotometrically at pH 7.4 and 282 nm ($\Delta E = 1{,}370\ M^{-1}cm^{-1}$). Assay mixtures contained 0.77 mM uridine, 88 mM potassium phosphate buffer (pH 7.4) and enzyme in a final volume of 3 ml in a quartz cuvette having a 1 cm light path. The reactions were continuously monitored with a Gilford recording spectrophotometer using a full scale recorder setting of 0.1 optical density units.

Purine nucleoside phosphorylase activity was monitored at 251 nm ($\Delta E = 1{,}500\ M^{-1}cm^{-1}$) and the reaction mixture concentrations of inosine and potassium phosphate (pH 8.0) were 0.16 mM and 250 mM, respectively.

One unit of each enzyme activity was defined as that amount of enzyme which catalysed the formation of one micromole of product per minute under the assay conditions described above.

*Cell Culture*

The media (550 l) for a culture of *Escherichia coli* B (ATCC 11303) contained the following components in g/l: 18.9 $Na_2HPO_4.7\ H_2O$, 6.3 $KH_2PO_4$, 0.2 $MgSO_4.7\ H_2O$, 2.0 $(NH_4)_2SO_4$, 0.5 adenosine, and 8.0 Difco vitamin-free casamino acids. These components were dissolved in water and the resulting solution was autoclaved. Cells were grown at 36°C with continuous aeration. When the culture reached stationary phase (17 hrs), the cells were harvested by centrifugation.

*Preparation of the Cell Extract*

The cell paste (2.5 kg) was suspended in potassium phosphate buffer (5 mM, 5 l), pH 8.0 and the cells were disrupted by means of a pressure cell. The supernatant fluid obtained upon centrifugation of the disrupted cell suspension was sorted in aliquots at −73°C.

*Treatment with Calcium Phosphate Gel*

Calcium phosphate gel was prepared as described in Keilin, D. and Hartree, E.F., Proc. Roy. Soc. Ser. B. 1938, *124*, 397, except that deionised water was used throughout the procedure. The dry weight of the gel suspension was 36 mg/ml. The gel (1 l) was slowly added to the thawed cell extract (3 l) (I in Tables I and II) at 3°C with continuous mechanical stirring. This suspension was then stirred for one hr and centrifuged at 13,000 x g for 10 minutes at 3°C. The pellet was discarded and gel (5 l) was slowly added to the supernatant fluid. After stirring for 1 hour at 3°C, this suspension was centrifuged at 4,000 x g for 10 minutes. The resulting gel pellet (B) contained the bulk of the uridine phosphorylase. The resulting supernatant fluid (6.2 l) (II—P in Table II) contained the bulk of the purine nucleoside phosphorylase.

*Further Purification of Uridine Phosphorylase*

Purification of this enzyme is summarised in Table I. All operations were performed at 3°C. Gel pellet B, described above, was suspended in potassium phosphate buffer (4 l, 20 mM) pH 7.4, (Buffer A). After gentle mechanical stirring for 1.5 hrs, the suspension was centrifuged at 4,000 x *g* for 10 minutes. The supernatant fluid was saved and the pellet extracted as above with Buffer A (2 l, 40 mM). The suspension was centrifuged and the extraction was then repeated with further Buffer A (2 l, 40 mM). To the combined supernatant fluids (9.845 l) (II—U) was slowly added enzyme grade (Schwarz/Mann) ammonium sulfate (2.36 kg) with mixing. After settling for 24 hrs, the precipitate was removed and discarded. Ammonium sulphate was added to the clear supernatant with mechanical stirring over a two hour period. After the precipitate had settled for 48 hrs, the precipitate was dissolved in Buffer A (20 mM) giving a final volume of 845 ml. This solution was dialyzed against Buffer A (20 mM, 20 l) for 22 hrs and then against Buffer A (20 mM, 29 l) for 4 hours.

DEAE-cellulose (Whatman microgranular DE—52)(240 g) was slowly added with stirring to the dialyzed preparation (III—U). The resin was washed with Buffer A (2 l) and then packed into a column. The final resin bed dimensions were 5 cm in diameter x 31 cm in height. The uridine phosphorylase activity was eluted from the column with a linear gradient (3 l) starting from 20 mM Buffer A and ending with 500 mM Buffer A. The fractions containing the bulk of the enzyme activity were pooled (690 ml) and this solution was dialyzed against Buffer A (5 mM, 25 l) for 18 hours and then against Buffer A (5mM, 21 l) for 4 hours.

The dialyzate (IV—U) (765 ml) was applied to an ECTEOLA-cellulose column, 5 cm in diameter

and 27 cm in height, which had been equilibrated with Buffer A (5 mM). After sample application, the column was washed with Buffer A (20 mM, 100 ml). The bulk of the enzyme activity was not retained by the column. Solid ammonium sulfate was added to the combined fractions having the highest activity (V—U) to a concentration of 3.0 M. The activity of the resulting suspension was stable when stored at 3°C.

TABLE I

Purification of Uridine Phosphorylase from *Escherichia coli* B

| | Step | Volume | Total Activity | Specific Activity | Purification | Recovery (overall) |
|---|---|---|---|---|---|---|
| | | 1 | Units | units /mg protein | -fold | % |
| I | Cell extract | 3.00 | 480,000 | 3.0 | 1 | 100 |
| II—U | Calcium phosphate gel eluate | 9.85 | 352,000 | 7.6 | 2.5 | 73 |
| III—U | Ammonium sulfate fraction | 1.10 | 248,000 | 7.5 | 2.5 | 52 |
| IV—U | DEAE-cellulose chromatography | 0.77 | 233,000 | 18.7 | 6.2 | 49 |
| V—U | ECTEOLA-cellulose chromatography | 0.57 | 229,000 | 31.4 | 10.5 | 48 |

*Further Purification of Purine Nucleoside Phosphorylase*

Purification of this enzyme is summarised in Table II. To the supernatant (II—P) from the calcium phosphate gel step (see above), DEAE-cellulose (Whatman microgranular DE52) (150 g) was slowly added with stirring at 3°C. After 30 minutes equilibration, the resin was separated from the fluid by means of a Buchner funnel equipped with a coarse sintered-glass bottom. The resin was then resuspended in Buffer A (10 mM) and poured into a column. After settling and washing with Buffer A (10 mM, 5 l), the resin bed had the dimensions 4.9 cm in diameter × 14.5 cm in height. The column was then eluted with a linear gradient (4 l) of Buffer A from 10 mM ot 1000 mM. The combined fractions (III—P) having the highest enzyme activity had a volume of 2110 ml. This solution was dialyzed against deionized water (10 l) at 3°C for 9 hrs. The dialyzate was applied to an ECTEOLA-cellulose (BioRad) column, 5 cm in diameter and 25 cm in height, which had been equilibrated at 25°C with potassium phosphate, pH 8.0, (Buffer B) (5 mM). The column was washed with Buffer B (5 mM, 1 l) and then eluted with a linear gradient (6 l) of Buffer B from 5 to 500 mM. The combined fractions having the highest enzyme activity (IV—P) had a volume of 2,470 ml. Solid ammonium sulfate was added to this solution to a concentration of 3.0 M. The enzyme was stable in this suspension at 3°C.

TABLE II

Purification of Purine Nucleoside Phosphorylase from *Escherichia coli* B

| | Step | Volume | Total Activity | Specific Activity | Purification | Recovery (overall) |
|---|---|---|---|---|---|---|
| | | l | Units | Units/mg protein | -fold | % |
| I | Cell extract | 3.00 | 53,370 | 0.34 | 1 | 100 |
| II—P | Calcium phosphate gel supernatant | 6.20 | 36,600 | 0.83 | 2.4 | 69 |
| III—P | DEAE-cellulose chromatography | 2.11 | 20,700 | 0.93 | 2.7 | 39 |
| IV—P | ECTEOLA-cellulose chromatography | 2.47 | 18,800 | 8.60 | 25.3 | 35 |

Example II — *2,6-Diamino-9-B-D-arabinofuranosylpurine*
(2,6-Diaminopurine arabinonucleoside)

The reaction mixture consisted of an aqueous suspension (1 l) which contained uracil arabinonucleoside (57 mmoles), 2,6-diaminopurine (118 mmoles), $K_2HPO_4$ (3 mmoles), sodium azide (3 mmoles), purine nucleoside phosphorylase (see Example I, 15,000 units) and uridine phosphorylase (see Example I, 8,000 units). The mixture was adjusted to pH 7.1 before addition of the enzymes. After 7 days at 37°C, the insoluble material in the reaction mixture was collected by centrifugation and extracted with water (700 ml) at 50°C. After 30 minutes of mechanical stirring, the suspension was allowed to settle at 3°C for 17 hrs. The insoluble material was collected by filtration and then suspended in anhydrous methanol (1.8 l). This suspension was gently boiled for one hr after which time the residue was collected by filtration and the filter cake washed with methanol (350 ml). The filter cake was then resuspended in methanol (350 ml) and boiled for one hr. After filtration and washing as above, the filter cake was dried *in vacuo* yielding 8.07 g of product, designated "first crop".

The filtrates from the above-described extractions were lyophilized and then returned to the supernatant fluid of the original reaction mixture. Additional uracil arabinonucleoside (44 mmoles) was added to this mixture. After 5 days at 37°C, the insoluble material present in the reaction mixture was extracted as described above, yielding 9.08 g of product, designated "second crop".

Anal. Calcd. for $C_{10}H_{14}N_6O_4$

| | | | |
|---|---|---|---|
| Theory: | C, 42.55; | H, 5.00; | N, 29.78 |
| 1st crop found: | C, 42.67; | H, 5.10; | N, 29.82 |
| 2nd crop found: | C, 42.67; | H, 5.03; | N, 29.65 |

$\lambda$max 0.5 N NaOH = 278,254

The overall yield was 60.7 mmoles representing a 51% yield of arabinonucleoside with respect to the amount of free base used and 60% with respect to the amount of uracil arabinonucleoside used.

Example III — *6-amino-9-B-D-arabinofuranosylpurine*
(Adenine arabinonucleoside)

The reaction mixture consisted of an aqueous suspension (1.18 l) which contained uracil arabinonucleoside (23.4 mmoles), adenine (22.5 mmoles), $K_2HPO_4$ (3 mmoles), sodium azide (0.3 mmoles), purine nucleoside phosphorylase (1580 units, see Example I) and uridine phosphorylase (820 units, see Example I). The suspension was adjusted to pH 7.3 before addition of enzymes. After 19 days at 37°C, the reaction mixture was maintained at 3°C for 17 hrs. The insoluble material was then collected by filtration and washed with water (200 ml). The filter cake was then suspended in water

(200 ml) and brought to the boil. After standing at 25°C for 17 hrs, the insoluble material was collected by filtration and resuspended in water (310 ml). This suspension was boiled for 20 minutes, allowed to stand for 7 hrs at 25°C and then for 17 hrs at 3°C. The precipitate was collected by filtration. After drying *in vacuo,* the product was obtained (2.51 g).

Anal. Calcd. for $C_{10}H_{13}N_5O_4 \cdot H_2O$

Theory: C, 42.11; H, 5.30; N, 24.55

Found: C, 42.23; H, 5.35; N, 24.62

UV Spectra: pH 7.0 $\lambda$max 260nm

$\lambda$max 228 nm

The overall yield was 9.4 moles representing a 41.8% yield of arabinonucleoside with respect to the amount of free base used and 40.2% with respect to the amount of uracil arabinonucleoside used.

## Claims

1. A process for producing 9—B—D-arabinonucleosides of purines characterised in that the process comprises reacting a purine base of formula (I)

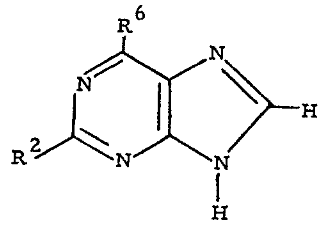

(I)

wherein a) $R^2$ is a hydrogen atom or an amino group and $R^6$ is a hydrogen or halogen atom or a hydroxyl, mercapto, amino, alkoxy, alkylthio, aryl -(lower)- alkylthio or an alkylamino group, wherein the term "alkyl" is denoted to mean an alkyl group having one to twelve carbon atoms in a straight or branched chain and wherein the term "(lower) alkyl" is defined as an alkyl group having one to seven carbon atoms in a straight or branched chain; or b) $R^2$ is a fluorine atom and $R^6$ is an amino group; or c) $R^2$ is a methylamino group and $R^6$ is an amino or methylamino group; with arabinose-l-phospohate in the presence of an arabinosyl-transferring enzyme system of the phosphorylase type, and recovering the purine arabinonucleoside of formula (II)

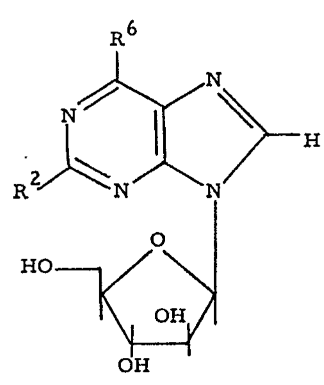

(II)

thus produced, wherein $R^2$ and $R^6$ are as hereinbefore defined, from the reaction medium.

2. A process as claimed in claim 1 characterised in that the arabinosyl-transferring enzyme system comprises at least one purine phosphorylase.

3. A process as claimed in claim 1 or claim 2 characterised in that the arabinose-l-phosphate is generated enzymatically *in situ* from an arabinosyl donor and inorganic phosphate.

4. A process as claimed in claim 3 characterised in that the arabinosyl donor is a purine

nucleoside, a pyrimidine nucleoside or a mixture thereof, or a mixture thereof with non-nucleosidic material.

5. A process as claimed in claim 3 or claim 4 characterised in that a pyrimidine nucleoside phosphorylase enzyme or a purine nucleoside phosphorylase enzyme is employed in the enzymatic generation of arabinose-l-phosphate.

6. A process as claimed in any preceding claim characterised in that the enzyme or enzymes are of microbial origin.

7. A process as claimed in claim 6 characterised in that the enzyme or enzymes are obtained from *B stearothermophilus* or *E coli* B strain, ATCC 11303.

8. A process as claimed in any preceding claim characterised in that it is carried out in aqueous medium or a medium containing up to 50% organic solvent.

9. A process as claimed in any preceding claim characterised in that the reaction mixture contains inorganic phosphate in the concentration range of 0.001 mM to 2000 mM, particularly 1 to 200 mM.

10. A process as claimed in any preceding claim characterised in that it is carried out in a medium having a pH of from 5 to 9, particularly 6.0 to 8.5.

11. A process as claimed in any preceding claim characterised in that it is carried out at a temperature of 3 to 70°C, particularly 25—45.

12. A process as claimed in any preceding claim characterised in that the purine arabinonucleoside is recovered by ion exchange chromatography or crystallisation.

**Revendications**

1. Procédé pour produire des 9—B—D-arabinonucléosides de bases puriques, caractérisé en ce que l'on fait réagir une base purique de formule (I):

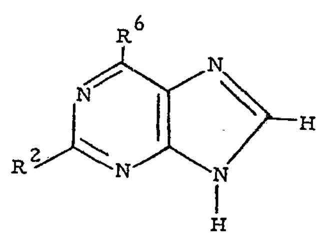

(I).

[dans laquelle a) $R^2$ est un atome d'hydrogène ou un groupe amino et $R^6$ est un atome d'hydrogène ou d'halogène ou un groupe hydroxyle, mercapto, amino, alcoxyle, alkylthio, arylalkylthio inférieur ou alkylamino, le terme "alkyle" étant destiné à désigner un groupe alkyle ayant 1 à 12 atomes de carbone en chaîne linéaire ou ramifiée et l'expression "alkyle (inférieur)" se définissant comme étant un groupe alkyle ayant 1 à 7 atomes de carbone en chaîne linéaire ou ramifiée; ou b) $R^2$ est un atome de fluor et $R^6$ est un groupe amino; ou c) $R^2$ est un groupe méthylamino et $R^6$ est un groupe amino ou méthylamino] avec de l'arabinose-l-phosphate en présence d'un système d'enzyme de transfert d'arabinosyle du type phosphorylase, et l'on recueille du milieu réactionnel l'arabinonucléoside de base purique de formule (II);

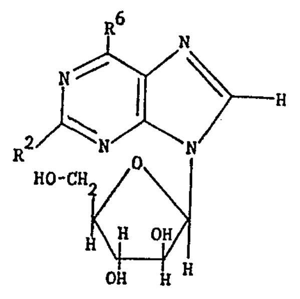

(II)

[dans laquelle $R^2$ et $R^6$ sont comme défini ci-dessus] ainsi produit.

2. Procédé selon la revendication 1, caractérisé en ce que le système d'enzyme de transfert d'arabinosyle comprend au moins une nucléoside purique-phosphorylase.

3. Procédé selon la revendication 1 ou la revendication 2, caractérisé en ce que l'arabinose-l-phosphate est engendré sur place par voie enzymatique à partir d'un donneur de groupe arabinosyle et d'un phosphate minéral.

4. Procédé selon la revendication 3, caractérisé en ce que le donneur d'arabinosyle est un nucléoside purique, un nucléoside pyrimidique ou un de leurs mélanges, ou un de leurs mélanges avec une matière non nucléosidique.

5. Procédé selon la revendication 3 ou la revendication 4, caractérisé en ce qu'on utilise une enzyme nucléoside pyrimidine-phosphorylase ou une enzyme nucléoside purique-phosphorylase pour la formation enzymatique de l'arabinose-l-phosphate.

6. Procédé selon l'une quelconque des revendications précédentes, caractérisé en ce que la ou les enzymes sont d'origine microbienne.

7. Procédé selon la revendication 6, caractérisé en ce que la ou les enzymes sont obtenues à partir d'une souche de *B stearothermophilus* ou *E coli* B, ATCC 11303.

8. Procédé selon l'une quelconque des revendications précédentes, caractérisé en ce qu'il est mis en oeuvre en milieu aqueux ou dans un milieu contenant jusqu'à 50% de solvant organique.

9. Procédé selon l'une quelconque des revendications précédentes, caractérisé en ce que le mélange réactionnel contient du phosphate minéral en une gamme de concentrations de 0,001 mM à 2 000 mM, notamment 1 à 200 mM.

10. Procédé selon l'une quelconque des revendications précédentes, caractérisé en ce qu'il est mis en oeuvre dans un milieu ayant un pH de 5 à 9, notamment de 6,0 à 8,5.

11. Procédé selon l'une quelconque des revendications précédentes, caractérisé en ce qu'il est mis en oeuvre à une température de 3° à 70°C, notamment de 25 à 45°C.

12. Procédé selon l'une quelconque des revendications précédentes, caractérisé en ce qu'on recueille l'arabinonucléoside de base purique par chromatographie d'échange d'ions ou cristallisation.

## Patentansprüche

1. Verfahren zur Herstellung der 9—B—D-Arabinonukleoside der Purine, dadurch gekennzeichnet, daß man eine Purin-Base der Formel (I)

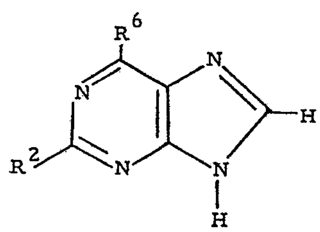

(I)

in der,

a) $R^2$ ein Wasserstoffatom oder eine Aminogruppe und $R^6$ ein Wasserstoff- oder Halogenatom oder eine Hydroxyl-, Mercapto-, Amino-, Alkoxyl-, Alkylthio-, Aryl-(niedrig)-Alkylthio- oder eine Alkylaminogruppe ist, worin "Alkyl" eine Alkylgruppe mit 1 bis 12 Kohlenstoffatomen in gerader oder verzweigter Kette und worin "(niedrig)-Alkyl" eine Alkylgruppe mit 1 bis 7 Kohlenstoffatomen in gerader oder verzweigter Kette; oder

b) $R^2$ ein Fluoratom und $R^6$ eine Aminogruppe; oder

c) $R^2$ eine Methylaminogruppe und $R^6$ eine Amino- oder Methylaminogruppe bedeuten;

mit Arabinosel-l-phosphat in Gegenwart eines Arabinosyl-Enzymtransfer-Systems des Phosphorylase-Typs zur Umsetzung bringt und das gebildete Purinarabinonukleosid der Formel (II)

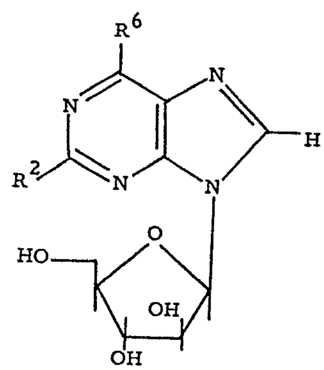

(II)

in der $R^2$ und $R^6$ die oben angegebenen Bedeutungen haben, aus dem Reaktionsmedium gewinnt.

2. Verfahren nach Anspruch 1, dadurch gekennzeichnet, daß das Arabinosyl-Enzymtransfersystem mindestens eine Purinnukleosidphosphorylase aufweist.

3. Verfahren nach Anspruch 1 oder 2, dadurch gekennzeichnet, daß das Arabinose-l-phosphate enzymatisch und in situ aus einem Arabinosyl-Donor und anorganischem Phosphat gebildet wird.

4. Verfahren nach Anspruch 3, dadurch gekennzeichnet, daß der Arabinosyl-Donor ein Purinnukleosid, ein Pyrimidinnukleosid oder ein Gemisch hiervon, oder ein Gemisch hiervon mit nichtnukleosidem Material ist.

5. Verfahren nach Anspruch 3 oder 4, dadurch gekennzeichnet, daß bei der enzymatischen Bildung des Arabinose-l-Phosphats ein Pyrimidinnukleosidphosphorylase-Enzym oder ein Purinnukleosidphosphorylase-Enzym verwendet wird.

6. Verfahren nach einem der Ansprüche 1 bis 5, dadurch gekennzeichnet, daß das oder die Enzyme mikrobischen Ursprungs ist.

7. Verfahren nach Anspruch 6, dadurch gekennzeichnet, daß das oder die Enzyme aus B Stearothermophilus oder E Coli-stamm B, ATCC 11303 erhalten werden.

8. Verfahren nach einem dem Ansprüche 1 bis 7, dadurch gekennzeichnet, daß es in wässrigem Medium oder in einem Medium durchgeführt wird, das bis zu 50% organisches Lösungsmittel enthält.

9. Verfahren nach einem der Ansprüche 1 bis 8, dadurch gekennzeichnet, daß das Reaktionsgemisch anorganisches Phosphat in einem Konzentrationsbereich von 0,001 mM bis 2000 mM, insbesondere 1 bis 200 mM, enthält.

10. Verfahren nach einem der Ansprüche 1 bis 9, dadurch gekennzeichnet, daß es in einem Medium mit einem pH von 5 bis 9, insbesondere 6,0 bis 8,5, durchgeführt wird.

11. Verfahren nach einem der Ansprüche 1 bis 10, dadurch gekennzeichnet, daß es bei einer Temperatur von 3 bis 70°C, insbesondere 25 bis 45°C durchgeführt wird.

12. Verfahren nach einem der Ansprüche 1 bis 11, dadurch gekennzeichnet, daß das Purinarabinonukleosid durch Ionenaustauschchromatographie oder Kristallisation gewonnen wird.